Europäisches Patentamt

European Patent Office

Office européen des brevets

(11) Numéro de publication: **0 260 186 B1**

(12) **FASCICULE DE BREVET EUROPEEN**

(45) Date de publication de fascicule du brevet: **04.03.92**

(51) Int. Cl.⁵: **A23K 1/00**, A23K 1/18, A61K 9/30, A61K 9/52

(21) Numéro de dépôt: **87401973.0**

(22) Date de dépôt: **03.09.87**

(54) **Nouvelles compositions pour l'enrobage des additifs alimentaires destinés aux ruminants et additifs alimentaires ainsi enrobés.**

(30) Priorité: **04.09.86 FR 8612412**

07-05-1986

(43) Date de publication de la demande:
**16.03.88 Bulletin 88/11**

(45) Mention de la délivrance du brevet:
**04.03.92 Bulletin 92/10**

(84) Etats contractants désignés:
**AT BE CH DE ES FR GB IT LI LU NL SE**

(56) Documents cités:
WO-A-84/00282      WO-A-87/03173
FR-A- 2 401 619     FR-A- 2 401 620
FR-A- 2 514 261     FR-A- 2 565 101
US-A- 4 181 710

CHEMICAL ABSTRACTS, vol. 104, no. 3, janvier 1986, page 422, résumé no. 18905z, Columbus, Ohio, US; & JP-A-60 141 243 (NIPPON SODA CO., LTD) 26-07-1985

CHEMICAL ABSTRACTS, vol. 107, no. 15, octobre 1987, page 617, résumé no. 133070x, Columbus, Ohio, US; & JP-A-61 88 844 (KYOWA HAKKO KOGYO CO., LTD)

(73) Titulaire: **RHONE-POULENC SANTE**
**20, avenue Raymond Aron**
**F-92160 Antony(FR)**

(72) Inventeur: **Ardaillon, Pierre**
**10, Impasse Beau-Vallon**
**F-69800 Saint-Priest(FR)**
Inventeur: **Autant, Pierre**
**14, rue de Pourcheroux**
**F-03600 Commentry(FR)**
Inventeur: **Bourrain, Paul**
**32/0 Chemin des Peupliers**
**F-69570 Dardilly(FR)**
Inventeur: **Cartillier, André**
**Champfromenteau**
**F-03600 Commentry(FR)**

(74) Mandataire: **Le Pennec, Magali et al**
**RHONE-POULENC SANTE, Service Brevets,**
**20 Avenue Raymond Aron**
**F-92165 Antony Cédex(FR)**

## Description

La présente invention concerne de nouvelles compositions pour l'enrobage des additifs alimentaires destinés aux ruminants qui sont stables dans un milieu dont le pH est égal ou supérieur à 5,5 et qui permettent la libération de l'additif alimentaire dans un milieu dont le pH est inférieur ou égal à 3,5.

Lorsqu'on administre à des ruminants certaines substances biologiquement actives (médicaments, vitamines, aminoacides), il se produit, lors du passage dans le rumen, une destruction enzymatique de ces substances favorisée par le temps de séjour (quelques heures à plusieurs jours) et par le pH (compris entre 5 et 6). Il en résulte que la substance active qui est dégradée perd la majeure partie de son efficacité lorsqu'elle arrive dans la caillette.

Il importe donc de pouvoir protéger ces substances biologiquement actives par des enrobages qui soient stables dans la panse des ruminants, c'est-à-dire qui soient stables à la dégradation par les microorganismes et qui permettent la libération des substances biologiquement actives dans une partie de l'appareil digestif, plus particulièrement dans la caillette, dont le pH est inférieur ou égal à 3,5. Alors que la durée de la protection dans la panse doit être relativement longue (quelques heures à plusieurs jours), la libération de la substance active dans la caillette doit s'effectuer en un temps relativement court (quelques minutes à 1 ou 2 heures).

De nombreuses compositions d'enrobage sont connues. Ainsi, dans le brevet français FR 81 18954 (2 514 261) a été décrit un enrobage constitué d'un copolymère sensible aux variations du pH, choisi parmi les copolymères du styrène avec les vinylpyridines, et d'un polymère non hydrosoluble insensible aux variations du pH, choisi parmi l'acétobutyrate de cellulose, l'éthylcellulose et le propionate de cellulose, ce polymère favorisant la libération de la substance active à un pH compris entre 1 et 2,5 et permettant de diminuer l'extractibilité de la substance active en milieu aqueux. Cependant, de tels enrobages, s'ils donnent des résultats satisfaisants avec la méthionine, conduisent à des résultats inférieurs avec la lysine dont les caractéristiques en particulier la solubilité dans l'eau, sont nettement différentes de celles de la méthionine.

Pour enrober des aliments destinés à la nourriture des ruminants, il a été proposé (brevet français FR 78 23966 (2 401 620) d'utiliser des copolymères styrène-vinylpyridine contenant des substances hydrophobes choisies parmi les acides gras contenant 10 à 32 atomes de carbone, des acides polycarboxyliques contenant 10 à 22 atomes de carbone par groupe carboxyle qui améliorent la protection en diminuant la suceptibilité globale de la pellicule d'enrobage aux milieux aqueux de caractère faiblement acide. Dans ces compositions, la substance hydrophobe est présente dans l'enrobage à raison de 5 à 75% de la masse de la substance polymère. Dans les granulés de substance active ainsi enrobée l'enrobage représente de 5 à 50% de la masse des granules. Généralement les granulés enrobés ont un diamètre compris entre 1,2 et 19 mm. Pour améliorer les propriétés des granulés enrobés, il est possible d'ajouter à la composition d'enrobage des charges lamellaires telles que la poudre d'aluminium le talc ou le mica.

Dans ces compositions, la teneur en copolymère aminé basique reste très élevée. Par ailleurs la composition d'enrobage ne paraît véritablement efficace que dans le cas de granulés de taille relativement importante dans lesquels la couche d'enrobage est assez épaisse (voisine de 150 microns).

Dans le cas de l'introduction de charges lamellaires, il est possible de se heurter à des problèmes technologiques de mise en oeuvre en particulier dans la technique du spray-coating.

Il est également décrit dans le brevet français FR 78 23749 (2 401 619) des granulés constitués d'un noyau de principe actif dont le pH a été neutralisé par addition d'agent neutralisant et qui est protégé par un enrobage à base du même mélange que celui utilisé dans le brevet précédemment cité. La protection n'est correcte que dans la mesure où l'agent de neutralisation est utilisé.

Il a maintenant été trouvé, et c'est ce qui fait l'objet de la présente invention, que l'on peut enrober efficacement des substances actives, en particulier la lysine et la méthionine, sans nécessiter l'usage d'agent de neutralisation, au moyen d'une composition constituée d'un copolymère aminé basique et d'une substance hydrophobe dont le point de fusion est supérieur à 60°C, la teneur en substance hydrophobe étant comprise entre 50 et 90% de la masse totale de l'enrobage.

La substance hydrophobe dont le point de fusion est supérieur à 60°C est choisie parmi les acides gras, les esters gras, les alcools gras et, éventuellement, leurs mélanges.

Les compositions d'enrobage selon l'invention, en dehors de la différence importante de comportement à pH = 6 et à pH = 2, présentent l'avantage de posséder une plus faible toxicité du fait de la faible quantité de copolymère aminé basique qu'elles contiennent. De plus, ces compositions présentent une excellente résistance à la dégradation au stockage (attrition, agglomération) ainsi qu'à la dégradation biologique due aux moisissures.

Dans la composition d'enrobage selon l'invention, le copolymère aminé basique est choisi parmi les copolymères du styrène avec les vinylpyridines telles que la vinyl-2 pyridine, la vinyl-4 pyridine ou la méthyl-2 vinyl-5 pyridine. Généralement, dans le copolymère aminé basique, la teneur en azote est comprise entre 3 et 14%.

Les substances hydrophobes dont le point de fusion est supérieur à 60°C sont, de préférence, l'acide stéarique et l'acide béhénique. D'un intérêt tout particulier est l'acide stéarique dont la pureté est supérieure à 90%.

Dans les compositions selon la présente invention, la substance hydrophobe, qui est en grand excès par rapport au copolymère basique assure une bonne rétention à pH = 6 et le copolymère basique joue le rôle de liant entre les cristaux de la substance hydrophobe. Le liant, qui est réparti de façon uniforme dans l'épaisseur de l'enrobant, se détruit, en milieu acide, sous l'action des protons entraînant ainsi une destruction de la cohésion membranaire avec libération rapide du principe actif.

Les compositions d'enrobage selon l'invention, en dehors du copolymère basique et de la substance hydrophobe, peuvent contenir des adjuvants dont le rôle est de faciliter la mise en oeuvre des techniques de préparation de ces compositions ou d'améliorer les caractéristiques physico-chimiques. Il peut être avantageux d'utiliser des agents plastifiants (triacétine, prolylèneglycol), des agents antistatiques (triglycérides à chaînes polyoxyéthylénées), des agents fongicides, des agents émulsifiants (esters de sorbitan oxyéthylénés, sucroglycérides), des agents de compatibilisation (gommes naturelles ou semi-naturelles comme les polysaccharides tels que les alginates, la gomme adragante, les pectines, les carraghénates, la gomme xanthane), des éthers cellulosiques (carboxyméthyl-, méthyl-, hydroxy-propyl-cellulose) ou des charges tels que des sels minéraux, du sucre, de l'amidon ou des protéines. Ces divers adjuvants peuvent représenter jusqu'à 3% en poids de la composition d'enrobage.

Les compositions selon l'invention peuvent être obtenues en dispersant ou en dissolvant le copolymère basique dans une solution ou une dispersion de la substance hydrophobe dans un solvant organique ou dans un mélange de solvants organiques convenables choisis en fonction de la nature spécifique des constituants. Généralement la composition d'enrobage est obtenue après évaporation du ou des solvants. Comme solvant organique, on utilise généralement un alcool (éthanol), un éther (tétrahydrofuranne), une cétone (acétone), ou un solvant aliphatique chloré (chlorure de méthylène, dichloro-1,2 éthane) ou un mélange de ces solvants. Il est particulièrement avantageux d'utiliser un mélange éthanol-dichloro-1,2 éthane, un mélange éthanol-chlorure de méthylène, un mélange éthanol-acétone ou un mélange éthanol-tétrahydrofuranne.

Les compositions d'enrobage selon la présente invention sont particulièrement utiles pour protéger des substances thérapeutiques ou nutritives diverses telles que des médicaments tels que le nitroxynil, des hormones peptidiques ou polypeptidiques, des vitamines telles que la vitamine A ou des amino-acides tels que la méthionine et la lysine destinés à être administrés par voie orale à des ruminants. Ces substances enrobées sont généralement mélangées à la nourriture des animaux.

Les compositions d'enrobage selon l'invention conviennent avantageusement pour l'enrobage de la méthionine et de la lysine.

Les substances enrobées sont de préférence des granulés sous forme de microcapsules constitués d'un noyau central entouré d'une pellicule continue de la composition d'enrobage. Cependant les substances actives peuvent aussi être dispersées dans la composition d'enrobage. En général, la composition d'enrobage représente 5 à 50% en poids du granulé ou de la dispersion.

La présente invention concerne également les substances biologiquement actives enrobées ou dispersées dans une composition d'enrobage décrite précédemment.

Les granulés peuvent être obtenus par application des techniques connues. Selon la nature de la composition d'enrobage, on utilise soit des techniques d'extrusion ou de pulvérisation de solutions ou d'émulsions en lit fluidisé, soit des techniques d'encapsulation en milieu fondu ou semi-fondu, soit des techniques d'enrobage en milieu liquide telle que la coacervation.

Les granulés obtenus selon la présente invention sont stables au stockage et lors des manipulations, ne se détériorent pas lors de la confection des aliments pour animaux et ne sont pas détruits lors de leur absorption par les animaux et en particulier par écrasement ou par broyage lors de la mastication. Pour augmenter la résistance des granulés au stockage à des températures relativement élevées (60°C), et plus particulièrement pour éviter le collage des grains entre eux, il est avantageux d'effectuer un pelliculage au moyen, par exemple, de zéine ou d'hydroxypropylméthylcellulose.

La taille des granulés sera fonction de l'utilisation qui doit être faite et plus particulièrement elle sera déterminée en fonction de l'animal auquel ils sont destinés. Les granulés de substance active à enrober peuvent avoir un diamètre compris entre 0,1 et 5 mm.

Généralement, il est suffisant que l'épaisseur de la couche enrobante soit voisine de 50 microns quelle que soit la taille du granulé pour obtenir un résultat satisfaisant. Il en résulte que le taux d'enrobant sera d'autant plus faible que la taille du granulé initial sera plus grande. Ainsi, si les granulés de substance active à enrober ont un diamètre moyen de 0,55 mm, le taux d'enrobant sera voisin de 35% alors que si les granulés à enrober ont un diamètre moyen de 1,1 mm, le taux d'enrobant sera voisin de 20%. En augmentant le diamètre initial des granulés, le taux de substance active dans le granulé enrobé est augmenté et le rapport polymère basique/substance active est diminué ce qui représente des avantages considérables tant sur le plan économique que sur celui de l'alimentarité.

Pour mettre en évidence la sensibilité des compositions d'enrobage selon l'invention aux variations du pH, des tests sont utilisés qui permettent de mesurer le relargage de la matière active en fonction du temps à différentes valeurs du pH et, notamment à pH = 6 et à pH = 2.

Par exemple, le relargage de la substance active présente dans les granulés est examiné en agitant, dans des conditions déterminées, une quantité connue de granulés dans le milieu tamponné maintenu à pH constant à une température de 40°C. On compare les vitesses de relargage d'un échantillon à différentes valeurs du pH et plus particulièrement à pH = 6 et à pH = 2.

Les exemples suivants, donnés à titre non limitatif, illustrent les compositions d'enrobage selon la présente invention ainsi que leur utilisation pour la préparation de matières actives enrobées.

### EXEMPLE 1

Selon la technique du lit fluidisé avec une cuve équipée d'un système WURSTER, on enrobe 350 g de méthionine, préablablement granulée sous formes de particules sphériques titrant 98% dont le diamètre moyen est compris entre 0,5 et 0,63 mm par une solution dont la composition est la suivante :
- acide stéarique (P.F. = 68-69°C; indice d'acide 194-198)     :     88 g
- copolymère vinyl-2 pyridine-styrène (70-30)     :     22 g
- dichloro-1,2 éthane     :     500 cm3
- éthanol     :     500 cm3
- antistatique (Labrasol, marque déposée GATTEFOSSE)     :     3 cm3

La viscosité du copolymère vinyl-2 pyridine-styrène (70-30) est de 0,560 mesurée à la concentration de 5 g/litre dans le diméthylformamide à 20°C.

La solution, maintenue à 27°C, est pulvérisée en 3 heures 40.

On obtient ainsi 392 g de granulés enrobés titrant 73% en méthionine.

Le relargage de la méthionine est déterminé en dispersant 8 g de granulés ainsi préparés dans 1 litre d'une solution tamponnée maintenue à 40°C et agitée magnétiquement.

La quantité de méthionine relarguée est contrôlée sur des prélèvements, les essais étant effectués à pH = 6 et à pH = 2.

Les résultats sont rassemblés dans le tableau 1.

### EXEMPLE 2

On opère comme dans l'exemple 1 mais en effectuant la pulvérisation en 1 heure 30.

On obtient ainsi 450 g de granulés titrant 71,6% en méthionine.

Les résultats des essais de relargage sont donnés dans le tableau 1.

### EXEMPLE 3

On opère comme dans l'exemple 1 mais en utilisant une composition d'enrobage contenant 48,8 g d'acide stéarique et 12,2 g de copolymère vinyl-2 pyridine-styrène (70-30) et en effectuant la pulvérisation en 1 heure à 47°C.

On obtient ainsi 400 g de granulés titrant 82% en méthionine.

Les résultats des essais de relargage sont donnés dans le tableau 1.

### EXEMPLE 4

On opère comme dans l'exemple 1 mais en utilisant une composition d'enrobage contenant 40 g d'acide stéarique et 10 g de copolymère vinyl-2 pyridine-styrène (70-30) et en effectuant la pulvérisation en 45 minutes à 45°C.

On obtient ainsi 381 g de granulés titrant 86% en méthionine.

Les résultats des essais de relargage sont donnés dans le tableau 1.

## EXEMPLE 5

On opère comme dans l'exemple 1 en remplaçant le dichloro-1,2 éthane par du chlorure de méthylène.

La solution, maintenue à 30°C, est pulvérisée en 44 minutes.

On obtient ainsi 448 g de granulés titrant 76,8% en méthionine.

Les résultats des essais de relargage sont donnés dans le tableau 1.

## EXEMPLE 6

On dissout 88 g d'acide stéarique (P.F. = 66,9°C; indice d'acide 199) (acide PRIFRAC commercialisé par UNICHEMA) et 22 g de copolymère vinyl-2 pyridine-styrène (70-30) dans 80 g de méthylisobutylcétone.

On chauffe cette solution à 85°C et l'ajoute en 8 minutes à une solution agitée au moyen d'une turbine du type Polytron de 990 cm3 d'eau et de 4,2 cm3 de soude à 10% (p/v). On obtient ainsi une émulsion qui est fluide et homogène à une température supérieure ou égale à 54°C.

Selon la technique du lit fluidisé avec une cuve équipée d'un système WURSTER, on enrobe 350 g de méthionine, préalablement granulée sous forme de particules sphériques titrant 98% dont le diamètre moyen est compris entre 0,5 et 0,63 mm par l'émulsion obtenue précédemment.

L'émulsion, maintenue à 64°C, est pulvérisée en 59 minutes.

On obtient ainsi 434 g de granulés enrobés titrant 78% en méthionine.

Les résultats des essais de relargage sont donnés dans le tableau 1.

## EXEMPLE 7

On opère comme dans l'exemple 6 mais en utilisant une émulsion obtenue par dispersion à 85°C d'une solution de 88 g d'acide stéarique et de 22 g de copolymère vinyl-2 pyridine - styrène (70-30) dans 80 g de méthylisobutylcétone dans 495 cm3 d'eau et 4,2 cm3 de soude à 10% (p/v).

L'émulsion, maintenue à 66°C, est pulvérisée en 33 minutes.

On obtient ainsi 440 g de granulés enrobés titrant 77,8% en méthionine.

Les résultats des essais de relargage sont donnés dans le tableau 1.

## EXEMPLE 8

On opère comme dans l'exemple 6 en remplaçant 80 g de méthylisobutylcétone par 80 g d'acétate de butyle.

L'émulsion, maintenue à 63°C, est pulvérisée en 65 minutes.

On obtient ainsi 436 g de granulés enrobés titrant 79% en méthionine.

Les résultats des essais de relargage sont donnés dans le tableau 1.

Tableau 1

| Exemple | Titre en méthionine % | % de méthionine relarguée à pH = 6 après | | % de méthionine relarguée à pH = 2 après | | |
|---|---|---|---|---|---|---|
| | | 6h | 24 h | 15 mn | 30 mn | 60 mn |
| 1 | 72,9 | 1,3 | 2,9 | 82,0 | 84,0 | 100 |
| 2 | 71, | 1,5 | 3,2 | 86,0 | 89,0 | 100 |
| 3 | 82,0 | 3,9 | 8,3 | 100 | | |
| 4 | 86,0 | 5,6 | 14 | 99,0 | 99,0 | 100 |
| 5 | 76,8 | 1,2 | 3,0 | 100 | 100 | 100 |
| 6 | 78,0 | 1,5 | 2,4 | 100 | 100 | 100 |
| 7 | 77,8 | 1,7 | 3,0 | 100 | 100 | 100 |
| 8 | 79,0 | 2,4 | 5,2 | 100 | 100 | 100 |

## EXEMPLE 9

On opère comme dans l'exemple 1 mais en remplaçant la méthionine par 350 g de chlorhydrate de lysine préalablement granulé sous forme de particules sphériques dont le diamètre moyen est compris entre 0,63 et 0,80 mm et titrant 82%. La durée de la pulvérisation est de 3 heures 30 à 27-30°C. On obtient ainsi 450 g de granulés titrant 60,0% en chlorhydrate de lysine.

Les résultats des essais de relargage sont donnés dans le tableau 2.

EXEMPLE 10

On opère comme dans l'exemple 9 mais en utilisant une composition d'enrobage contenant 93,5 g d'acide stéarique et 16,5 g de copolymère vinyl-2 pyridine-styrène (70-30) et en effectuant la pulvérisation en 3 heures 15 à 28-30°C.

On obtient ainsi 380 g de granulés titrant 57,6% en chlorhydrate de lysine.

Les résultats des essais de relargage sont donnés dans le tableau 2.

EXEMPLE 11

On opère comme dans l'exemple 9 mais en utilisant une composition d'enrobage contenant 99 g d'acide stéarique et 11 g de copolymère vinyl-2 pyridine-styrène (70-30) et en effectuant la pulvérisation en 3 heures 15 à 29-35°C.

On obtient ainsi 445 g de granulés titrant 60,2% en chlorhydrate de lysine.

Les résultats des essais de relargage sont donnés dans le tableau 2.

EXEMPLE 12

On opère comme dans l'exemple 9 mais en utilisant une composition d'enrobage contenant 77 g d'acide stéarique et 33 g de copolymère vinyl-2 pyridine-styrène (70-30) et en effectuant la pulvérisation en 1 heure 50 à 32-40°C.

On obtient ainsi 460 g de granulés titrant 58,4% en chlorhydrate de lysine.

Les résultats des essais de relargage sont donnés dans le tableau 2.

EXEMPLE 13

On opère comme dans l'exemple 9 mais en utilisant une composition d'enrobage contenant 60,5 g d'acide stéarique et 49,5 g de copolymère vinyl-2 pyridine-styrène (70-30) et en effectuant la pulvérisation en 2 heures 10 à 39-41°C.

On obtient ainsi 460 g de granulés titrant 59,8% en chlorhydrate de lysine.

Les résultats des essais de relargage sont donnés dans le tableau 2.

EXEMPLE 14

On opère comme dans l'exemple 9 mais en effectuant la pulvérisation en 1 heure 30 à 37-39°C.

On obtient ainsi 455 g de granulés titrant 61% en chlorhydrate de lysine.

Les résultats des essais de relargage sont donnés dans le tableau 2.

EXEMPLE 15

On opère comme dans l'exemple 9 mais en utilisant une solution de composition enrobante dans du tétrahydrofuranne pur et en effectuant la pulvérisation en 2 heures à 20°C.

On obtient ainsi 457 g de granulés titrant 60,7% en chlorhydrate de lysine.

Les résultats des essais de relargage sont donnés dans le tableau 2.

EXEMPLE 16

On opère comme dans l'exemple 9 mais en utilisant une solution de composition enrobante dans un mélange éthanol-tétrahydrofuranne (1-1 en volumes) et en effectuant la pulvérisation en 1 heure 40 à 20°C.

On obtient ainsi 457 g de granulés titrant 62% en chlorhydrate de lysine.

Les résultats des essais de relargage sont donnés dans le tableau 2.

EXEMPLE 17

On opère comme dans l'exemple 9 mais en utilisant une solution de composition enrobante dans un mélange éthanol-acétone (1-1 en volumes) et en effectuant la pulvérisation en 3 heures 30 à 40°C.

On obtient ainsi 455 g de granulés titrant 59,2% en chlorhydrate de lysine.

Les résultats des essais de relargage sont donnés dans le tableau 2.

EXEMPLE 18

On opère comme dans l'exemple 9 en remplaçant l'acide stéarique par l'acide béhénique (acide docosanoïque; P.F. = 76°C), le mélange dichloroéthane-éthanol par un mélange tétrahydrofuranne-éthanol et en effectuant la pulvérisation en 2 heures 40 à 56-59°C.

On obtient ainsi 445 g de granulés titrant 59,2% en chlorhydrate de lysine.

Les résultats des essais de relargage sont donnés dans le tableau 2.

EXEMPLE 19

On opère comme dans l'exemple 9 mais en utilisant une composition d'enrobage ne contenant pas d'agent antistatique.

La solution, maintenue à 35°C, est pulvérisée en 1 heure 40 minutes.

On obtient ainsi 456 g de granulés enrobés titrant 60% en chlorhydrate de lysine.

Les résultats des essais de relargage sont donnés dans le tableau 2.

EXEMPLE 20

On opère comme dans l'exemple 9 mais en utilisant une solution d'enrobage dans laquelle le dichloro-1,2 éthane est remplacé par la même quantité de chlorure de méthylène.

La solution, maintenue à 35°C, est pulvérisée en 42 minutes.

On obtient ainsi 455 g de granulés enrobés titrant 59% en chlorhydrate de lysine.

Les résultats des essais de relargage sont donnés dans le tableau 2.

EXEMPLE 21

On utilise une émulsion préparée comme dans l'exemple 6 pour enrober 350 g de chlorhydrate de lysine préalablement granulé sous forme de particules sphériques dont le diamètre moyen est compris entre 0,63 et 0,80 mm et titrant 82%.

L'émulsion, maintenue à 65°C, est pulvérisée en 64 minutes.

On obtient ainsi 451 g de granulés enrobés titrant 57% en chlorhydrate de lysine.

Les résultats des essais de relargage sont donnés dans le tableau 2.

EXEMPLE 22 (exemple comparatif)

On opère comme dans l'exemple 9 en utilisant une composition d'enrobage constituée de 93,5 g d'acide stéarique technique et de 16,5 g de copolymère vinyl-2 pyridine-styrène (70-30).

On obtient ainsi des granulés enrobés titrant 60,0% en chlorhydrate de lysine.

Les résultats des essais de relargage sont donnés dans le tableau 2.

L'acide stéarique technique utilisé, dont le point de fusion est de 56°C, a la composition suivante :

acides saturés =
$C_{18}$ acide stéarique : 44,1%
$C_{17}$ : 2%
$C_{16}$ acide palmitique : 47%
$C_{14}$ acide myristique : 3%
$C_{12}$ acide laurique : traces
acides insaturés =
$C_{18}$ acide oléique
$C_{16}$ acide palmitoléique 4,5%
$C_{14}$ acide myristoléique

EXEMPLE 23 (exemple comparatif)

7

On opère comme dans l'exemple 9 en utilisant une composition d'enrobage contenant 99 g d'acide stéarique technique et 11 g de copolymère vinyl-2 pyridine-styrène.

On obtient ainsi des granulés enrobés titrant 61,3% en chlorhydrate de lysine.

Les résultats des essais de relargage sont donnés dans le tableau 2.

Des exemples 22 et 23, il ressort que l'utilisation d'acide stéarique technique ne permet pas d'avoir une bonne rétention à pH = 6 comparativement, en particulier, aux produits des exemples 10 et 11.

Tableau 2

| Exemples | Titre en chlorhydrate lysine % | % de chlorhydrate de lysine relargué à pH = 6 après | | % de chlorhydrate de lysine relargué à pH = 2 après | | |
|---|---|---|---|---|---|---|
| | | 6 h | 24 h | 15 mn | 30 mn | 60 mn |
| 9 | 60,0 | 6,0 | 15,6 | 98 | 98 | 100 |
| 10 | 57,6 | 5,0 | 13,0 | 100 | 100 | 100 |
| 11 | 60,2 | 23,0 | 37,0 | 100 | 100 | 100 |
| 12 | 58,4 | 6,5 | 14,0 | 100 | 100 | 100 |
| 13 | 59,8 | 9,3 | 16,6 | 97,0 | 97,0 | 99 |
| 14 | 61,0 | 6,0 | 11,5 | 100 | 100 | 100 |
| 15 | 60,7 | 13,7 | 28,3 | 100 | 100 | 100 |
| 16 | 62,0 | 8,0 | 17,0 | 100 | 100 | 100 |
| 17 | 59,2 | 13,3 | 23,4 | 100 | 100 | 100 |
| 18 | 59,2 | 9,0 | 17,0 | 100 | 100 | 100 |
| 19 | 60,0 | 2,4 | 6,1 | 100 | 100 | 100 |
| 20 | 59,0 | 6,6 | 18,4 | 100 | 100 | 100 |
| 21 | 57,0 | 5,8 | 9,5 | 100 | 100 | 100 |
| 22 | 60,0 | 98 | 98 | 86 | 86 | 98 |
| 23 | 61,3 | 76 | 88 | 98 | 98 | 98 |

L'efficacité in vivo des compositions d'enrobage selon l'invention peut être mise en évidence dans le test suivant :

Des échantillons de granulés enrobés (environ 0,5 g) sont introduits dans des sachets en nylon ayant une maille de 300 x 300 microns. Les sachets sont placés pendant 48 heures dans le rumen de brebis fistulées. Les sachets sont alors récupérés et lavés. On utilise 3 ou 4 brebis par produit. La quantité de substance active présente dans les sachets est déterminée par une méthode appropriée.

Les résultats obtenus sont rassemblés dans le tableau 3.

Tableau 3

| Exemples | Substance active | Rapport acide stéarique/ copolymère | % de substance active extraite après 48 h |
|---|---|---|---|
| A | | | 5 |
| 1 B | Méthionine | 80/20 | 0 |
| C | | | 0 |
| I | | | 11 |
| J | Chlorydrate de | 80/20 | 1 |
| 5 K | lysine | | 7 |
| L | | | 2 |
| M | | | 0 |
| 6 N | Chlorydrate de | 85/15 | 0 |
| O | lysine | | 11 |
| P | | | 5 |

EXEMPLE 24

Selon la technique de "spray-coating", on enrobe 350 g de granulés contenant de l'acétate de vitamine A dont le diamètre est compris entre 0,5 et 0,8 mm et dont la composition en poids est la suivante :

| | |
|---|---|
| acétate de vitamine A | 32,2% |
| gélatine | 40,9% |
| lactose | 9,2% |
| glycérine | 6,6% |
| stabilisants | 11,1% |
| par une solution d'enrobage ayant la composition suivante : | |
| acide stéarique (PRIFRAC 9553) | 114,8 g |
| copolymère vinyl-2 pyridine - styrène (70-30) | 28,7 g |
| éthanol | 650 cm3 |
| dichloro-1,2 éthane | 650 cm3 |

La solution, maintenue à 36°C, est pulvérisée à un débit de 10 cm3/minute.

On obtient ainsi des granulés enrobés dont le titre exprimé en vitamine A est de 618000 UI/g, les granulés de départ ayant un titre de 845000 UI/g.

Le relargage in vitro est déterminé à 37°C dans un milieu gastrique artificiel à pH = 1,2 en présence de pepsine (selon USP XX).

Après 2 heures, le pourcentage de produit actif, relargué est de 93% pour les granulés enrobés et est de 86% pour les granulés de départ.

Après 48 heures, la résistance au rumen est de 86,0 ± 3,2% pour les granulés enrobés et est de 3,0 ± 2,2% pour les granulés de départ.

## Revendications

1. Composition pour l'enrobage d'une substance biologiquement active, destinée aux ruminants, ladite composition étant stable dans un milieu dont le pH est supérieur ou égal à 5 et permettant la libération de la substance biologiquement active dans un milieu dont le pH est inférieur à 3,5, caractérisée en ce qu'elle est constituée d'un copolymère aminé basique et d'une substance hydrophobe, dont le point de fusion est supérieur à 60°C, la teneur en substance hydrophobe étant comprise entre 50 et 90 % de la masse totale de l'enrobage; ladite composition étant non associée a un agent de neutralisation et la teneur en substance hydrophobe étant non égale à 50 %, celleci excluant les sels d'acides gras.

2. Composition selon la revendication 1 caractérisée en ce que le copolymère aminé basique est choisi parmi les copolymères des vinyl-pyridines avec le styrène dans lesquels la teneur en azote est comprise entre 3 et 14 %.

3. Composition selon la revendication 2 caractérisée en ce que le copolymère aminé basique est choisi parmi les copolymères de la vinyl-2 pyridine, vinyl-4 pyridine et méthyl-2 vinyl-5 pyridine avec le sryrène.

4. Composition selon la revendication 1 caractérisée en ce que la substance hydrophobe dont le point de fusion est supérieur à 60°C est choisie parmi les acides gras, les esters gras, les alcools gras et leurs mélanges.

5. Composition selon la revendication 4 caractérisée en ce que la substance hydrophobe est choisie parmi l'acide stéarique et l'acide béhénique.

6. Composition selon la revendication 4 caractérisée en ce que la substance hydrophobe est un acide stérique dont la pureté est supérieure à 90 %.

7. Composition selon la revendication 1 caractérisée en ce qu'elle contient en outre des adjuvants choisis parmi les antistatiques, les fongicides, les émulsifiants, des agents de compatibilisation, des éthers cellulosiques, des sels minéraux, de l'amidon ou des protéines, ces adjuvants représentant jusqu'à 3% en poids de la composition d'enrobage.

**8.** Granulés destinés à être administrés par voie orale à des animaux caractérisés en ce qu'ils sont constitués d'un noyau contenant la substance biologiquement active entouré par une composition d'enrobage selon l'une des revendications 1 à 7.

**9.** Granulés selon la revendication 8 caractérisés en ce que la composition d'enrobage représente entre 5 et 50% en poids du granulé enrobé.

**10.** Granulés selon l'une des revendications 8 ou 9 caractérisés en ce que la substance active est choisie parmi des médicaments, des hormones, des vitamines et des amino-acides.

**11.** Granulés selon l'une des revendications 8 à 9 caractérisés en ce que la substance active est choisie parmi la méthionine et la lysine.

**Claims**

**1.** Composition for coating a biologically active substance intended for ruminants, the said composition being stable in a medium with a pH greater than or equal to 5 and enabling the biologically active substance to be released in a medium with a pH less than 3.5, characterised in that it consists of a basic amino copolymer and a hydrophobic substance with a melting point greater than 60°C, the hydrophobic substance content being between 50 and 90% of the total mass of the coating, the said composition not being associated with a neutralising agent and the hydrophobic substance content not being equal to 50%, the latter excluding fatty acid salts.

**2.** Composition according to claim 1, characterised in that the basic amino copolymer is chosen from amongst copolymers of vinylpyridines with styrene in which the nitrogen content is between 3 and 14%.

**3.** Composition according to claim 2, characterised in that the basic amino copolymer is chosen from amongst copolymers of 2-vinylpyridine, 4-vinylpyridine and 2-methyl-5-vinyl-pyridine with styrene.

**4.** Composition according to claim 1, characterised in that the hydrophobic substance with a melting point greater than 60°C is chosen from amongst fatty acids, fatty esters and fatty alcohols and the mixtures thereof.

**5.** Composition according to claim 4, characterised in that the hydrophobic substance is chosen from amongst stearic acid and behenic acid.

**6.** Composition according to claim 4, characterised in that the hydrophobic substance is a stearic acid with a purity greater than 90%.

**7.** Composition according to claim 1, characterised in that it additionally contains adjuvants chosen from amongst antistatic agents, fungicides, emulsifiers, compatibility-inducing agents, cellulose ethers, inorganic salts, starch or proteins, these adjuvants representing up to 3% by weight of the coating composition.

**8.** Granules intended for oral administration to animals, characterised in that they consist of a nucleus containing the biologically active substance surrounded by a coating composition according to one of claims 1 to 7.

**9.** Granules according to claim 8, characterised in that the coating composition represents between 5 and 50% by weight of the coated granule.

**10.** Granules according to either of claims 8 or 9, characterised in that the active substance is chosen from amongst medicaments, hormones, vitamins and amino acids.

**11.** Granules according to one of claims 8 and 9, characterised in that the active substance is chosen from amongst methionine and lysine.

10

**Patentansprüche**

1. Masse zum Umhüllen einer für Wiederkäuer bestimmten biologisch aktiven Substanz, welche Masse in einem Milieu, dessen pH-Wert größer oder gleich 5 ist, stabil ist und die Freisetzung der biologisch aktiven Masse in einem Milieu, dessen pH-Wert kleiner als 3,5 ist, zuläßt, dadurch gekennzeichnet, daß sie aus einem basischen, aminhaltigen Copolymer und einer hydrophoben Substanz gebildet ist, deren Schmelzpunkt höher als 60°C ist, wobei der Gehalt an hydrophober Substanz zwischen 50 und 90 % der gesamten Umhüllungsmasse liegt, die Masse nicht mit einem Neutralisationsmittel assoziiert ist und der Gehalt an hydrophober Substanz nicht gleich 50 % ist und Fettsäuresalze ausgeschlossen sind.

2. Masse nach Anspruch 1, dadurch gekennzeichnet, daß das basische aminhaltige Copolymer ausgewählt ist aus den Copolymeren von vinylpyridinen mit Styrol, worin der Stickstoffgehalt 3 bis 14 % beträgt.

3. Masse nach Anspruch 2, dadurch gekennzeichnet, daß das basische aminhaltige Copolymer ausgewählt ist aus den Copolymeren von 2-Vinylpyridin, 4-Vinylpyridin und 2-Methyl-5-vinylpyridin mit Styrol.

4. Masse nach Anspruch 1, dadurch gekennzeichnet, daß die hydrophobe Substanz, deren Schmelzpunkt höher als 60°C liegt, ausgewählt ist aus den Fettsäuren, den Fettsäureestern, den Fettalkoholen und ihren Mischungen.

5. Masse nach Anspruch 4, dadurch gekennzeichnet, daß die hydrophobe Substanz ausgewählt ist aus Stearinsäure und Behensäure.

6. Masse nach Anspruch 4, dadurch gekennzeichnet, daß die hydrophobe Substanz eine sterische Säure mit einer Reinheit größer als 90 % ist.

7. Masse nach Anspruch 1, dadurch gekennzeichnet, daß sie außerdem Zusatzstoffe, ausgewählt aus den antistatischen Mitteln, Fungiziden, Emulgiermitteln, Kompatibilisierungsmitteln, Celluloseäthern, anorganischen Salzen, Stärke oder Proteinen, enthält, wobei diese Zusatzstoffe bis zu 3 Gew.-% der Umhüllungsmasse ausmachen.

8. Granulate zur oralen Verabreichung an Tiere, dadurch gekennzeichnet, daß sie aus einem die biologisch aktive Substanz enthaltenden Kern, umgeben von einer Umhüllungsmasse nach einem der Ansprüche 1 bis 7, bestehen.

9. Granulate nach Anspruch 8, dadurch gekennzeichnet, daß die Umhüllungsmasse 5 bis 50 Gew.-% dem umhüllten Granulats ausmacht.

10. Granulate nach einem der Ansprüche 8 oder 9, dadurch gekennzeichnet, daß die aktive Substanz ausgewählt ist aus Medikamenten, Hormonen, Vitaminen und Aminosäuren.

11. Granulate nach einem der Ansprüche 8 bis 9, dadurch gekennzeichnet, daß die aktive Substanz ausgewählt ist aus Methionin und Lysin.